# EUROPÄISCHE PATENTANMELDUNG

(11) **EP 1 288 295 A2**
(43) Veröffentlichungstag der Anmeldung: **05.03.2003**
(21) Anmeldenummer: 02016913.2
(22) Anmeldetag: 31.07.2002
(51) Int. Cl.: C12N 15/09

(54) **Einsatz mutierter Erkennungssequenzen für mehrfache aufeinander folgende Rekombinase-vermittelte Rekombinationen in einem genetischen System**

(30) Priorität: 16.08.2001 DE 10140030
(71) Anmelder: GSF-Forschungszentrum für Umwelt und Gesundheit GmbH, 85764 Oberschleissheim (DE)
(72) Erfinder: Altmann, Markus Dr., 81543 München (DE); Neuhierl, Bernhard, 80689 München (DE); Hammerschmidt, Wolfgang Dr., 80686 München (DE)
(74) Vertreter: Reinhard - Skuhra - Weise & Partner

(57) **Zusammenfassung**

Die vorliegende Erfindung betrifft ein verbessertes Rekombinationsverfahren für die ortsspezifische Rekombinase-vermittelte Rekombination unter Verwendung von mutierten Erkennungssequenzen. Dabei wird von einem nicht identischen Paar von Erkennungssequenzmutanten Gebrauch macht. Jeder der Erkennungssequenzmutanten besteht aus zwei Erkennungssequenzen, die von einem Spacer getrennt sind. In die eine Erkennungssequenz wurden Mutationen eingeführt, so daß nach der Rekombination durch eine sequenzspezifische Rekombinase eine Erkennungssequenzmutante entsteht, die nicht mehr von der Rekombinase erkannt wird.

## Beschreibung

Die vorliegende Erfindung betrifft ein verbessertes Rekombinationsverfahren für die ortsspezifische Rekombination unter Verwendung von mutierten Erkennungssequenzen.

Die ortsspezifische Rekombination ist ein attraktives Werkzeug für die Manipulation von genetischen Systemen. Leider ist die Anzahl möglicher Rekombinationsreaktionen innerhalb einer einzelnen Zelle oder eines genetischen Systems beschränkt, weil jede Rekombinase nur einmal verwendet werden kann und die Anzahl der bekannten ortsspezifischen Rekombinasen beschränkt ist.

Eine von diesen ist beispielsweise die Cre-Rekombinase aus dem E. coli Bakteriophagen P1, die die ortsspezifische Rekombination zwischen zwei identischen loxP-Motiven in einer intramolekularen oder intermolekularen Weise vermittelt. Die Rekombinase Cre des E. coli Bakteriophagen P1 ist eine ortsspezifische Rekombinase, die eine DNA-Neuordnung über seine DNA-Zielsequenz, nämlich loxP, vermittelt (1). Die loxP-Sequenzen bestehen aus einer 8 bp Spacer Region, die von zwei 13 bp invertierten Wiederholungen (inverted repeats) flankiert wird, die als Erkennungssequenzen für die DNA-Bindung von Cre dienen (2,3). Das Rekombinationsereignis ist lediglich von diesen beiden Bestandteilen abhängig und wird mit absoluter Zuverlässigkeit durchgeführt. Es wurde erkannt, dass das Cre-loxP-System, genauso wie das Flp-FRT System von *S. cerevisiae* Rekombinationsereignisse in prokaryontischen ebenso wie in eukaryontischen Zellen wirkungsvoll katalysiert, einschließlich derjenigen aus Hefe, Pflanzen, Insekten und Säugetieren. Ortsspezifische Rekombinationssysteme werden in großem Umfang als Werkzeuge für konditionelle genetische Veränderungen in einzelnen Zellen und Tieren verwendet (bezüglich eines aktuellen Rückblicks siehe (4, 5) und die darin genannten Bezugnahmen).

Es existiert eine Vielzahl von weiteren ortsspezifischen Rekombinationssystemen, die auf einem Zweikomponenten-System basieren. Allen derartigen Systemen ist gemein, daß sie spezifische, sich wiederholende DNA Sequenzen aufweisen. Diese Sequenzen bestehen jeweils aus zwei Erkennungssequenzen, die durch einen Spacer voneinander getrennt sind und zueinander invers repetitiv sind. Die beiden Komponenten sind hierbei identisch. Neben den bereits oben genannten Beispielen sind noch das Zygosaccharomyces rouxii pSR1-, das Resolvase-rfsF- und das Phagen Mu Gin Rekombinase-System bekannt.

Die Rekombinase-Systeme, wie z.B. das Cre-loxP-System, können zur Exzision, Inversion oder Insertion von erkennungssequenz-flankierten DNA-Segmenten verwendet werden, weil die Rekombinasen sowohl intramolekulare (Exzision oder Inversion) als auch intermolekulare (Insertion) Rekombinationsereignisse vermittelt. Bei einer Exzision wird der Bereich einer DNA-Sequenz zwischen zwei Erkennungssequenzen herausgeschnitten. Ebenso ist es möglich, zirkuläre DNA, in der z.B. eine loxP-Sequenz vorhanden ist, in einen genetischen Locus, in dem ebenfalls eine loxP-Sequenz vorhanden ist, einzubringen. Hierbei ist zu beachten, dass nie nur die gewünschte (Hin-)reaktion stattfindet, sondern auch stets eine Rückreaktion.

Die Eigenschaften der Rekombinationssysteme, z.B. des Cre-Systems, wurden mit verschiedenen herkömmlichen Gen Targeting- und Verdrängungs-Strategien (4, 5) kombiniert. Üblicherweise beruhen herkömmliche genomische Veränderungen auf der zielgerichteten Integration eines modifizierten Allels. In eukaryotischen und prokaryotischen Zellen wird das Integrationsereignis durch homologe Rekombination innerhalb von Regionen erreicht, die das interessierende Allel flankieren. Ein positiver genetischer Marker ist zur Selektion von homologen Rekombinationsereignissen obligat, die in den meisten genetischen Systemen recht selten vorkommen. Es ist deswegen oftmals wünschenswert, diesen Marker in einem nachfolgenden Schritt zu entfernen, vorzugsweise in Verbindung mit dem verbleibenden Wild-Typ Allel. Zur Entfernung des Markergens (oder von DNA-Segmenten, die deletiert werden sollen), ermöglichen eingebaute loxP-Sequenzen die effiziente Exzision des loxP-flankierten DNA-Segments in einer strikt Cre-abhängigen Weise. Das ausgeschnittene Fragment wird zirkularisiert und geht durch Abbau verloren, jedoch verbleibt eine einzelne loxP-Sequenz im modifizierten Gen-Ort. Diese loxP-Sequenz kann später jedoch wieder zusammen mit einer weiteren loxP-Sequenz als eine weitere Stelle für die Rekombinase Cre dienen, wodurch es zu unerwünschten Rekombinationsereignissen kommen kann.

Eine genetische Manipulation von E. coli Plasmiden mit Inserten, die 100 kbp überschreiten, ist ein ziemlich neuer Ansatz, der durch das Klonieren von großen chromosomalen Fragmenten in Single Copy E. coli-Plasmide auf Grundlage des F-Faktors oder Bakteriophagen P1-Replikons, die BAC und PAC-Plasmide genannt werden, eingeführt wurde (6-8). Die synthetische Konstruktion von E. coli-Plasmiden einer derartigen Größe ist ebenfalls möglich (9,10) ebenso wie das molekulare Klonieren des vollständigen Herpesvirus-Genoms, das 250 kbps überschreitet und für bis zu 200 unterschiedliche Gene kodiert (für einen aktuellen Rückblick siehe Ref 11). Eine genetische Manipulation von BACs und PACs wird im allgemeinen durch unterschiedliche homologe Rekombinationsschemen in E. coli erreicht, was die Anwendung eines Selektions-Markergens (11,12) erforderlich macht. Vielfache unabhängige Veränderungen in einem einzigen Plasmid machen die unmittelbare Entfernung des selektierbaren Markers oder die nachfolgende Verwendung unterschiedlicher Markergene erforderlich. Ungefähr ein halbes Dutzend unterschiedlicher Antibiotika-Resistenzgene sind erhältlich (und eine noch größere Anzahl von auxotrophen Markern), jedoch kann deren effiziente Exzision nur durch eine relativ kleine Anzahl von ortsspezifischen Rekombinationssystemen erreicht werden, von denen die Kombinationen CreloxP, Flp-FRT, Resolvase-rfsF am besten bekannt sind. Als Folge hiervon ist die Anzahl der unabhängigen Veränderungen in einem DNA-Molekül ziemlich beschränkt.

Um das Repertoire genetischer Manipulationen mit Rekombinase-Systemen innerhalb einer einzelnen Zelle zu erhöhen, sind z.B. bei dem Cre-loxP-System Mutationen der Spacer Region (13) oder der invertierten Wiederholungen bzw. Inverted Repeats (14) durchgeführt worden, die die Eigenschaften von loxP-Sequenzen verändern. Eine loxP-Sequenz mit einer veränderten Spacer-Region kann nur mit einer äquivalenten oder gepaarten loxP-Sequenz rekombinieren, ist jedoch für eine Cre-vermittelte Rekombination mit einem Wild-Typ loxP-Ort oder einer anderen loxP-Variante nicht in der Lage (15). Nach Cre-vermittelter Rekombination kann die sich ergebende loxP-Sequenz noch durch Cre erkannt werden. Als Folge können loxP-Sequenzen mit veränderten Spacer-Regionen nicht für aufeinander folgende Rekombinationsereignisse im selben genetischen System verwendet werden.

Es wurde berichtet, dass loxP-Varianten mit veränderten Inverted Repeat-Regionen die stabile Integration eines loxP-flankierten DNA-Segments in einen einzelnen vorexistierenden loxP-Ort in einem Pflanzenchromosom begünstigen (14). Das Rekombinationsereignis hat die Erzeugung einer mutierten loxP-Sequenz mit Veränderungen in beiden Inverted Repeats und eine zweite loxP-Site zur Folge, die der Wild-Typ ist (14). Es wurde angenommen, dass die mutierte loxP-Sequenz ein schlechtes Substrat für die Cre-Rekombinase ist (14), jedoch wurde berichtet, dass das System als Ganzes durchlässig und instabil ist (16), d.h. es wurde von einer verbliebenen Eigenschaft der loxP-Mutante berichtet, weiterhin von Cre als Zielsequenz erkannt zu werden.

Der vorliegenden Erfindung liegt daher die Aufgabe zugrunde, ein verbessertes Rekombinationsverfahren zu schaffen, um vielfache und gerichtete Mutationen in einem genetischen System während vielfacher aufeinander folgender homologer und Rekombinasevermittelter Rekombinationsereignisse zu ermöglichen. Diese Aufgabe wird durch die in den unabhängigen Patentansprüchen dargelegten Merkmale gelöst. Bevorzugte Ausführungsformen und Weiterbildungen der Erfindung ergeben sich aus den Unteransprüchen.

Die oben genannte Aufgabe wird durch das erfindungsgemäße Rekombinationsverfahren gelöst, das von einem nicht identischen, Paar von Erkennungssequenzmutanten Gebrauch macht. Jeder der Erkennungssequenzmutanten besteht aus zwei Erkennungssequenzen, die von einem Spacer getrennt sind. In die eine Erkennungssequenz wurden Mutationen eingeführt, die andere entspricht dem Wildtyp. Nach der Rekombination durch eine sequenzspezifische Rekombinase entsteht dann eine Erkennungssequenzmutante, die in beiden Erkennungssequenzen Mutationen trägt und somit nicht mehr von der Rekombinase erkannt wird. Sie kann also nicht mehr für eine weitere Rekombinase-vermittelte Rekombination verwendet werden. Die normalerweise im Gleichgewicht mit der Hinreaktion stehende Rückreaktion (siehe oben) wird dadurch nicht mehr ausgeführt. Die Reaktion wird dadurch gerichtet. Weiterhin können die urprünglich bereitgestellten Erkennungssequenzmutanten (die jeweils nur eine mutierte Erkennungssequenz aufweisen) mehrmals in einem genetischen Systemen verwendet werden, da nach erfolgter Rekombination keine Sequenz mehr vorhanden ist, die mit weiteren eingeführten Erkennungssequenzmutanten (weder mit mutierten Sequenzen noch mit Wildtypsequenzen) konkurrieren könnte.

Dadurch besteht die Möglichkeit, ein DNA-Fragment mit höherer Effizienz als bisher in ein genetisches System einzubringen, bzw. eine unbegrenzte Anzahl von Abschnitten in einem genetischen System zu rekombinieren.

Erfindungsgemäß werden zwei nicht identische Erkennungssequenzmutanten verwendet, die in jeweils einer Erkennungssequenz Mutationen tragen, die zueinander invers repetitiv sind, wobei die jeweils andere Erkennungssequenz dem Erkennungssequenz-Wildtyp entspricht, um damit zwei oder mehrere aufeinander folgende Rekombinationsereignisse durch die Rekombinase in einem einzelnen genetischen System zu bewirken.

Der Begriff "Erkennungssequenzmutante", wie er im vorliegenden Zusammenhang verwendet wird, umfasst in den Wildtypsequenzen auftretende Mutationen der folgenden Art:
Punktmutationen eines Nukleotids oder weniger benachbarter Nukleotide, mehrere Nukleotide betreffende Mutationen, Deletionen, Additionen und Nukleotidaustausch.

Gemäß einer bevorzugten Ausführungsform handelt es sich bei den beiden nicht identischen Erkennungssequenzmutanten um loxP-Mutanten und zwar um die beiden loxP-Mutanten lox 66 und lox 71, entsprechend den Seq. Id.-Nr. 1 und 6. Weitere loxP-Erkennungssequenzmutanten weisen die Seq. Id. Nr. 2-5 und 7-10 auf.

Das erfindungsgemäße Rekombinationsverfahren zur Durchführung mehrfacher Rekombinationen mittels einer Rekombinase in einem einzelnen genetischen System, umfasst im einzelnen die folgenden Schritte:
Zunächst werden zwei nicht identische Erkennungssequenzmutanten in dem genetischen System bereitgestellt. "Genetisches System" bedeutet hierin beispielsweise eine prokaryontische oder eukaryontische Zelle oder auch einen Tier- oder Pflanzenorganismus. Beispiele für prokaryonte Systeme sind E. coli, Salmonella species, Bacillusspezies, Bakteriophagen. Eukaryonte Systeme sind beipielsweise humane und animale Zellen und Zellinien somatischer Natur, Maus, Zebrafisch, Drosophila, S. cerevisiae, Xenopus laevis.

Die beiden nicht identischen Erkennungssequenzmutanten tragen jeweils in einer Erkennungssequenz (die im Wildtyp als "inverted repeat"-Sequenzen bezeichnet werden) Mutationen, die zueinander invers repetitiv sind. Mit anderen Worten ist die eine mutierte Erkennungssequenz eines Erkennungssequenzmutanten invers repetitiv zu der mutierten Erkennungssequenz des anderen Erkennungssequenzmutanten. Unter invers repetitiven Sequenzen versteht man im allgemeinen direkt oder nicht direkt benachbarte DNA- und RNA-Sequenzelemente mit einer gegenläufig komplementären oder nahezu komplementären Sequenz. Die Sequenzen bilden dann so genannte inverted repeat-Sequenzen.

Die weitere in den Mutanten enthaltene Sequenz entspricht der Wildtypsequenz. Beispielsweise entspricht bei beiden loxP-Mutanten jeweils die andere Erkennungssequenz dem nicht mutierten loxP-Wildtyp. Die beiden loxP-Mutanten müssen so ausgerichtet sein, daß ihre Wildtyp-Sequenzen auf der dem Rekombinationsort zugewandten Seite liegen. Diese werden dann im Zuge eines Rekombinationsereignisses mit der entsprechenden DNA-Sequenz herausgeschnitten, wodurch dann eine loxP-Mutante zurückbleibt, die aus zwei mutierten Erkennungssequenzen besteht. Diese kann dann erfindungsgemäß nicht mehr von der Rekombinase Cre erkannt werden und ist somit nicht mehr in weitere, Cre-vermittelte Rekombinationsereignisse involviert.

Als nächster Schritt erfolgt bei dem erfindungsgemäßen Rekombinationsverfahren die Induktion einer sequenzspezifischen Rekombinase zur Durchführung eines Rekombinationsereignisses, wobei - wie oben angesprochen - eine Erkennungssequenzmutante in der durch Rekombination veränderten Nukleinsäuresequenz zurückbleibt, wobei diese Erkennungssequenzmutante nicht mehr von der Rekombinase erkannt wird. Dieses Verfahren kann dann beliebig oft zur Durchführung weiterer Rekombinationsereignisse wiederholt werden.

Gemäß einer bevorzugten Ausführungsform kommen bei dem erfindungsgemäßen Rekombinationsverfahren die beiden loxP-Mutanten lox 66 und lox 71 zum Einsatz. Die Orientierung dieser, wie auch aller anderen erfindungsgemäßen Erkennungssequenzmutanten ist eindeutig durch die sich zwischen den beiden Erkennungssequenzen befindliche Spacer-Sequenz vorgegeben (gegensinnig oder gleichsinnig). Ein Rekombinationsereignis kann im allgemeinen nur bei einer gleichsinnigen Ausrichtung der beiden Erkennungssequenzmutanten auftreten. Wichtig ist, daß die jeweiligen Wildtypsequenzen auf der dem Rekombinationsort zugewandten Seite liegen, das heißt, daß z.B. die loxP-Mutanten in der Reihenfolge: mutierte lox 66-Sequenz -> Wildtyp-lox 66-Sequenz -> Wildtyp-lox 71-Sequenz -> mutierte lox 71-Sequenz (oder umgekehrt) angeordnet sein müssen. Die hierin offenbarten Nukleinsäuresequenzen sind stets in 5' -> 3'-Richtung angeordnet.

Gemäß einer Ausführungsform sind bei dem erfindungsgemäßen Rekombinationsverfahren die Erkennungssequenzmutanten der Seq. Id. Nr. 1-5 in 5' -> 3'-Richtung von den Sequenzen ATTCC und TCTCG, die Erkennungssequenzmutanten der Seq. Id. Nr. 6-10 in 5'-> 3'-Richtung von den Sequenzen GCTTC und CTCTT flankiert.

Die Cre-Rekombinase kann z.B.durch Expression in dem genetischen System erzeugt werden, beispielsweise mittels eines Vektors, der die Rekombinase Cre kodiert.

Wie bereits oben angesprochen, kann es sich bei dem genetischen System der vorliegenden Erfindung um eine prokaryontische oder eukaryontische Zelle handeln, beispielsweise um eine Bakterien-, Hefe-, Pflanzen-, Insekten- oder Säugetierzelle. Genauso gut kann das genetische System jedoch aus einer definierten, isolierten Nukleinsäureeinheit, beispielsweise einem Plasmid, bestehen.

Das im Zuge das erfindungsgemäßen Rekombinationsverfahrens stattfindende Rekombinationsereignis kann aus einer Insertion oder Exzision einer DNA-Sequenz bestehen.

Beispielsweise kann bei einer Exzision eine DNA-Sequenz, die ein Markergen enthält, herausgeschnitten werden. Bei diesen Markergenen handelt es sich vorzugsweise um Antibiotikaresistenzgene, die beispielsweise eine Resistenz gegen Chloramphenicol, Tetracyclin oder Ampicillin verleihen.

Beispielsweise kann bei der Insertion eines DNA Segments, das auf einem mobilen genetischen Element wie einem extrachromosomalen Plasmid vorliegen kann, folgende Ausgangssituation bestehen: Das DNA Segment kann links von einer lox66 und rechts von einer lox71-Erkennungssequenz flankiert sein. Die Anordnung der beiden lox Varianten ist gleichsinnig. Auf dem zu inserierenden DNA Segment befindet sich auch ein geeignetes Markergen neben dem Gen oder genetischem Element von Interesse. Vorzugsweise liegt auch eine einzelne lox66 Erkennungssequenz bereits im Chromosom einer Zelle als Zielsequenz vor. Nach Expression von Cre kommt es zur Ausbildung der folgenden Struktur von rechts nach links: lockP - inseriertes DNA Segment - lox66. Damit ist die Rückwärtsreaktion, d.h. die Exzision des inserierten DNA Segments durch die blockierte lockP Erkennungssequenz unmöglich. Die noch vorhandene lox66 Erkennungssequenz kann für weitere Insertionen verwendet werden.

Das erfindungsgemäße Rekombinationsverfahren arbeitet am zuverlässigsten, wenn bei der Durchführung wiederholter Rekombinationsereignisse die nach dem ersten Rekombinationsereignis entstandene loxP-Mutante dieselbe Orientierung aufweist wie die zur Durchführung weiterer Rekombinationsereignisse bereitgestellten loxP-Mutanten, d.h. bezüglich der Spacer gleichsinnig ist. Zum Begriff der Orientierung gelten die oben dargelegten Ausführungen bezüglich lox 66 und lox 71 analog.

Die vorliegende Erfindung wird nachfolgend durch Beispiele sowie die beigefügten Figuren genauer erläutert.

In den Figuren zeigen:
**Figur 1:**
   Gerichtete Nukleotid-Sequenzen von Wildtyp (loxP) und mutierten (lox66/ lox71) loxP-Sequenzen. Flankierende Regionen, Inverted Repeats und die Spacer Region sind durch Leerstellen getrennt. Die Zahlen zeigen die Positionen der Nukleotide innerhalb der Inverted Repeats an. Die mutierten Nukleotide sind durch kleinere Buchstaben dargestellt.
**Figur 2:**
   Häufigkeit von Cre-vermittelten Rekombinationsereignissen. Cre-Rekombinase wurde vorübergehend über Nacht bei 30°C in E. coli exprimiert, das entweder das Plasmid p2724 oder p2725 trug. Die Zellen wurden geerntet und die Plasmide wurden durch Standardverfahren isoliert. Zehn pg jeder Plasmid-Zubereitung wurden in E. coli transformiert, die auf LB-Agar Platten, die Ampicillin enthielten, plattiert wurden. Die Kolonien wurden auf LB-Platten, die entweder Ampicillin (Amp), Chloramphenicol (Cm) oder Tetrazyklin (Tc) enthielten Replika-plattiert. Das Wachstum auf dieser Kombination von drei Antibiotika zeigte an, dass keine Rekombination aufgetreten war. Eine Resistenz gegen Ampicillin und Chloramphenicol, jedoch eine Empfindlichkeit gegenüber Tetrazyklin stimmt mit der Cre-vermittelten Rekombination zwischen lox66 und lox71 überein, jedoch nicht mit lockP. Jeder andere Phänotyp zeigt unerwünschte Rekombinationsereignisse. Die Häufigkeit der Phänotypen der Replika-plattierten Kolonien in Gegenwart unterschiedlicher Antibiotika ist angezeigt. Insgesamt wurden 208 Kolonien für jedes Test-Plasmid untersucht.

### Beispiele:

Mit dem nachfolgenden Testsystem wird dargestellt, dass spezielle Mutationen innerhalb der Inverted Repeats von loxP zu mutierten loxP-Sequenzen führen, die effizient rekombinieren, jedoch nach einer einzelnen Runde einer Cre-vermittelten Rekombination einen beständigen loxP-Ort bilden.

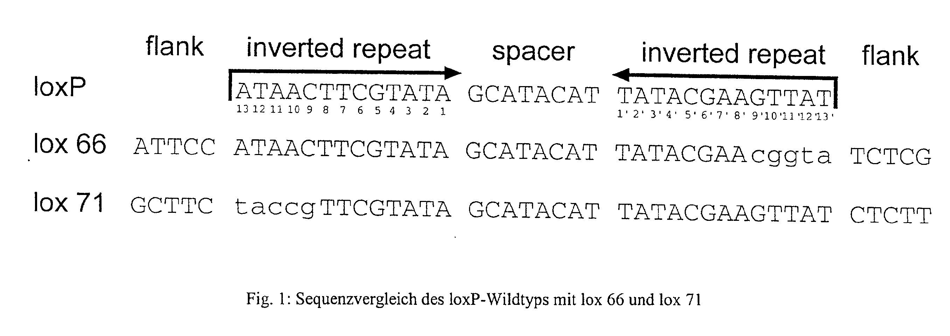

Die Klonierung des loxP-Testsystems basiert auf pACYC184. Das Tetrazyklin Resistenzgen wurde mit *Hind*III und *Ava*I ausgeschnitten, gefolgt von einer Insertion eines Fragments, das zwei mutierte loxP-Sequenzen, nämlich lox 66 und lox 71 enthielt (Fig. 1). Das DNA-Fragment, das lox 66 und lox 71 enthielt, wurde nach Annealing von zwei teilweise überlappenden Oligonukleotiden (5'-GGGAAGCTTCTACCGTTCGTATAGCATACATTA-TACGAAGTTATCTCTTGCGGGATATCGTCCATTCC-3' und 5'-CCCCCGAGATACCG-TTCGTATAATGTATGCTATACGAAGTTATGGAATGGACGATATCCCGCAAGAG-3'), nach Klenow-Enzym vermittelter Synthese eines doppelsträngigen DNA-Fragmentes und Verdauung mit *Hind*III und *Ava*I erzeugt. Dieses Plasmid wurde p2627 genannt.

Im nächsten Schritt wurde ein *Eco*47III-Fragment, dass das Tetrazyklin-Resistenzgen vom Plasmid pACYC184 enthielt, in die einzige *Eco*RV-Erkennungssequenz zwischen den beiden mutierten loxP-Sequenzen von p2627 eingefügt, wodurch p2632 erzeugt wurde. Dieses Plasmid wurde mit *Ase*I und teilweise mit *Pvu*II gespalten. Das Fragment, dass das Tetrazyklin-Resistenzgen, flankiert von den beiden mutierten loxP-Orten zusammen mit dem Chloramphenicol-Resistenzgen enthielt, wurde in pUC 19 eingefügt, das *Nde*I und *Sma*I verdaut wurde. Das sich ergebende Plasmid wurde p2722 genannt und trägt drei Antibiotika-Resistenzgene, eines flankiert von den mutierten loxP-Orten.

E. coli Zellen, die p2722 tragen, wurden mit einem zweiten Plasmid, nämlich p2676 transfiziert, das sich über den temperaturempfindlichen Ursprung von pSC101(17) repliziert und Cre ebenso wie ein Kanamycin-Resistenzgen kodiert. Eine Vermehrung der Zellen in Gegenwart von Kanamycin bei 30°C über Nacht führte zur Cre vermittelten Entfernung des Tetrazyklin-Resistenzgens im residenten Plasmid p2722, gefolgt von Vermehrung von Tetrazyklin empfindlichen Kolonien bei 42°C, die zum Verlust von p2676 führt. Das sich ergebende Plasmid p2723 trägt nunmehr den rekombinanten und mutierten loxP-Ort, der lockP genannt wird, was durch DNA-Sequenzierung bestätigt wurde.

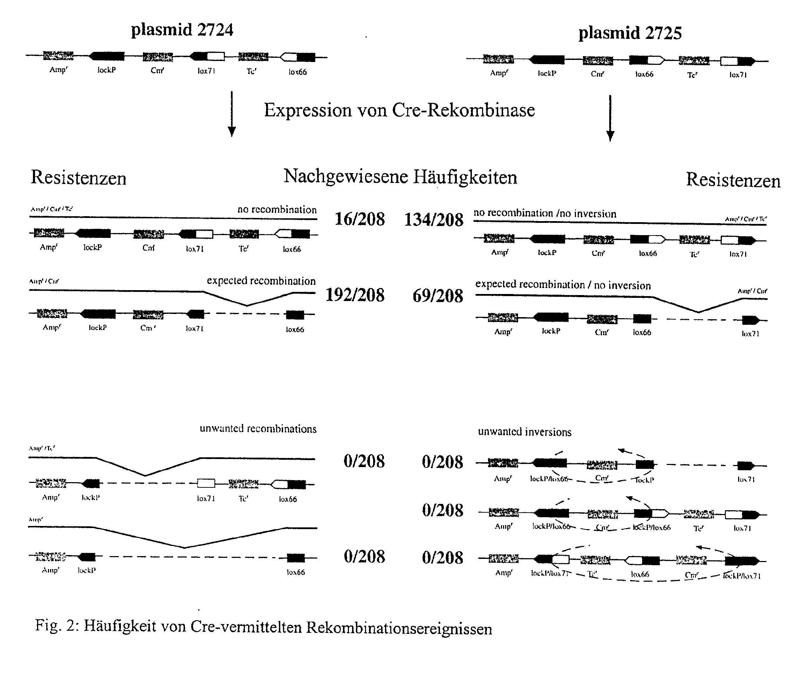

Die beiden endgültigen Testplasmide wurden durch Einfügen eines *Ase*I/*Ava*I-Fragmentes in den in gleicher Weise modifizierten *Bam*HI Ort von p2723 hergestellt, wobei das Fragment aus p2632 stammt, das durch ein Klenow-Enzym stumpf gemacht wurde. Das p2632 abgeleitete *Ase*I/*Ava*I-Fragment enthält die beiden mutierten loxP-Sequenzen lox26 und lox71, die das Tetrazyklin-Resistenzgen von pACYC184 flankiert. Die beiden endgültigen Plasmide, nämlich p2724 und p2725 (Fig. 2) tragen das lox66/lox71 flankierte Tetrazyklin-Resistenzgen in beiden möglichen Orientierungen bezüglich der lockP-Stelle und die Orte, die eine Resistenz gegen Chloramphenicol und Ampicillin kodieren (Fig. 2). Beide Test-Plasmide p2724 und p2725 (Fig. 2) tragen den identischen Satz von drei Antibiotika-Resistenzgenen, unterscheiden sich jedoch bezüglich der relativen Ausrichtung der drei loxP-Varianten lox66, lox71 und lockP. In p2724 sind alle drei loxP-Orte in derselben Richtung ausgerichtet, wohingegen in p2725 die lox66 und lox71 Orte und das Tetrazyklin-Resistenzgen dazwischen bezüglich des lockP-Ortes auf Grundlage ihrer 8 bp Spacer Sequenzen invertiert sind (Fig. 1). E. coli Zellen, die entweder p2724 oder p2725 tragen, wurden mit einem Expressionsplasmid, das Cre (p2676) kodiert transformiert, das ebenfalls Resistenz gegen Kanamyzin bereitstellt und über einen temperaturempfindlichen Ursprung der DNA-Replikation repliziert. Eine Stunde nach DNA-Transformation und phänotypischer Expression bei 30°C (die permissive Temperatur für p2676) wurde Kanamyzin und Ampicillin der flüssigen Kultur zugesetzt und die Zellen wurden weiterhin für 16 Stunden inkubiert. Plasma-DNA wurde erzeugt und 10 pg wurden in E. coli Stamm DH5α durch Standardverfahren transfiziert (18). Die Zellen wurden bei 37°C auf LB-Platten, die Ampicillin enthielten, über Nacht ausplattiert. Die Ampicillinresistenten Zellen wurden auf Replika-Platten, die Ampicillin, Kombinationen von Ampicillin/Tetrazyklin, Ampicillin/Chloramphenicol und Ampicillin/Tetrazyklin/-Chloramphenicol enthielten nach einer Inkubation über Nacht bei 37°C untersucht. Die Anzahl der Kolonien auf den verschiedenen Replika-Platten in Gegenwart unterschiedlicher Antibiotika gab einen ersten Hinweis auf die Verwendbarkeit von loxP-Stellen, die durch Cre rekombiniert wurden (Fig. 2).

Im Falle beider Test-Plasmide würden Kolonien, die in Gegenwart aller drei Antibiotika wachsen, keine Rekombination durchgemacht haben. Alternativ könnten derartige Kolonien modifizierte Test-Plasmide mit invertierten DNA-Segmenten zwischen den unterschiedlichen loxP-Orten enthalten. Kolonien, die in Gegenwart von Ampicillin und Chloramphenicol, jedoch nicht in Gegenwart von Tetrazyklin wuchsen, rekombinerten vermutlich wie angenommen zwischen lox66 und lox71, jedoch nicht über lockP. Der Verlust einer Chloramphenicol-Resistenz oder sowohl der Chloramphenicol als auch der Tetrazyklin Resistenz würde anzeigen, dass der lockP-Ort in die Cre-vermittelte Rekombinationsereignisse involviert war, die eine unerwünschte Rekombination zur Folge hatten (Fig. 2).

8 aus Experimenten mit dem Test-Plasmid p2724 oder p2725 gewonnene Kolonien, die das erwartete phänotypische Muster (Chloramphenicol und Ampicillin resistent, Tetrazyklin empfindlich) zeigten und 48 Kolonien aus Plasmid 2725, die wie durch ihren Phänotyp angezeigt (Chloramphenicol, Ampicillin und Tetrazyklin resistent) keine Rekombination durchgemacht hatten, wurden weiterhin durch Restriktions-Enzymanalyse untersucht. Alle Plasmid DNAs aus den unabhängigen Kolonien zeigten das vorhergesagte Restriktionsmuster (Daten nicht dargestellt) wie es von ihren Phänotypen erwartet wurde, bestimmt durch Replika-Plattieren. In keinem Fall hat sich eine Inversion von DNA-Segmenten zwischen den verschiedenen loxP-Orten herausgestellt. 5 von dem Test-Plasmid p2725 abstammende Klone mit einem unerwarteten Resistenzmuster litten unter einer vollständigen Umordnung von p2725, die nicht durch die Cre-vermittelte Verwendung irgend einer der mutierten loxP-Sequenzen erklärt werden kann (Daten nicht dargestellt). In keinem Fall war die mutierte lockP-Sequenz ein Substrat von Cre.

Zusammengenommen zeigt unsere Untersuchung, dass eine Cre-vermittelte Rekombination zwischen zwei mutierten loxP-Orten, nämlich lox66 und lox71 im Falle der direkt angeordneten loxP-Orte in p2724, effizient abläuft. Es ist nicht erkennbar, warum eine Cre-vermittelte Rekombination im Test-Plasmid p2725 weniger effizient ist. In diesem Plasmid liegen die loxP-Sequenzen in zwei Orientierungen vor, die die rekombinatorische Aktivität von Cre stören könnten. Noch wichtiger war in allen Fällen die sich ergebende lockP-Sequenz vollständig gegen Cre-vermittelte Rekombination beständig, was anzeigt, dass lockP funktionell als Ergebnis einer vorhergehenden ortsspezifischen Cre-Rekombination inaktiviert wurde.

Dieses Ergebnis war aus zwei Gründen überraschend. Zuerst wurde berichtet, dass die selben Kemorte lox 66 und lox 71 (Fig. 1 mit unterschiedlichen Nukleotiden, die die loxP-Motive flankieren) ein Substrat für Cre sein sollen, jedoch nur mit einer reduzierten Leistungsfähigkeit der Wildtyp loxP. Die lox66 und lox71 Orte erhielten nichtsdestotrotz ungefähr ein Fünftel ihrer rekombinatorischen Aktivität nach der Bildung des lockP-Motivs (14) bei. Zweitens zeigte die Proteinstruktur von Cre zusammen mit biochemischen Bindungsexperimenten, dass die Positionen 2, 3, 6 und 7 von loxP (Fig. 1) für die Cre-Bindung an loxP am wichtigsten waren, wohingegen Positionen jenseits von 9 nicht von größerer Bedeutung für die Cre-Bindung an ihr Zielmotiv (19,20) erschienen.

Die beiden loxP-Sequenzen, die in diesem Beispiel untersucht wurden, haben ihre Rekombinations-Leistungsfähigkeit nach ortspezifischer Rekombination vollständig verloren. Deswegen sind vielfach aufeinander folgende, durch loxP-Cre-vermittelte Rekombinationsereignisse in einer einzelnen Zelle oder sogar einem einzelnen DNA-Molekül möglich.

### Literatur:

1. Sternberg, N., Austin, S., Hamilton, D. and Yarmolinsky, M. (1978) Analysis of bacteriophage P1 immunity by using lambda-P1 recombinants constructed in vitro. *Proc Natl Acad Sci U* S *A,* 75, 5594-5598.
2. Mack, A., Sauer, B., Abremski, K. and Hoess, R. (1992) Stoichiometry of the Cre recombinase bound to the lox recombining site. *Nucleic Acids Res,* 20, 4451-4455.
3. Hoess, R., Abremski, K., Irwin, S., Kendall, M. and Mack, A. (1990) DNA specificity of the Cre recombinase resides in the 25 kDa carboxyl domain of the protein. *J Mol Biol,* 216, 873-882.
4. Dymecki, S.M. (2000) Site-specific recombination in cells and mice. In Joyner, A. L. (ed.), *Gene targeting -* a *practical approach.* 2nd Ed. Oxford University Press, Oxford, pp. 37-99.
5. Torres, R.M. and Kühn, R. (1997) *Laboratory protocols for conditional gene targeting,* Oxford University Press, Oxford.
6. Shizuya, H., Birren, B., Kim, U.J., Mancino, V., Slepak, T., Tachiiri, Y. and Simon, M. (1992) Cloning and stable maintenance of 300-kilobase-pair fragments of human DNA in Escherichia coli using an F-factor-based vector. *Proc Natl Acad Sci U* S *A,* 89, 8794-8797.
7. Ioannou, P.A., Amemiya, C.T., Garnes, J., Kroisel, P.M., Shizuya, H., Chen, C., Batzer, M.A. and de Jong, P.J. (1994) A new bacteriophage P1-derived vector for the propagation of large human DNA fragments. *Nat Genet,* 6, 84-89.
8. Shepherd, N.S., Pfrogner, B.D., Coulby, J.N., Ackerman, S.L., Vaidyanathan, G., Sauer, R.H., Balkenhol, T.C. and Sternberg, N. (1994) Preparation and screening of an arrayed human genomic library generated with the P1 cloning system. *Proc Natl Acad Sci U* S *A,* 91, 2629-2633.
9. O'Connor, M., Peifer, M. and Bender, W. (1989) Construction of large DNA segments in Escherichia coli. *Science,* 244,1307-1312.
10. Kempkes, B., Pich, D., Zeidler, R. and Hammerschmidt, W. (1995) Immortalization of human primary B-lymphocytes *in vitro* with DNA. *Proc Natl Acad Sci U* S *A,* 92, 5875-5879.
11. Brune, W., Messerle, M. and Koszinowski, U.H. (2000) Forward with BACs: new tools for herpesvirus genomics. *Trends Genet*, 16, 254-259.
12. Muyrers, J.P., Zhang, Y., Testa, G. and Stewart, A.F. (1999) Rapid modification of bacterial artificial chromosomes by ET-recombination. *Nucleic Acids Res,* 27, 1555-1557.
13. Lee, G. and Saito, I. (1998) Role of nucleotide sequences of loxP spacer region in Cremediated recombination. *Gene,* 216, 55-65.
14. Albert, H., Dale, E.C., Lee, E. and Ow, D.W. (1995) Site-specific integration of DNA into wild-type and mutant lox sites placed in the plant genome. *Plant J,* 7, 649-659.
15. Sauer, B. (1996) Multiplex Cre/lox recombination permits selective site-specific DNA targeting to both a natural and an engineered site in the yeast genome. *Nucleic Acids Res,* 24, 4608-4613.
16. Araki, K., Araki, M. and Yamamura, K. (1997) Targeted integration of DNA using mutant lox sites in embryonic stem cells. *Nucleic Acids Res,* 25, 868-872.
17. Chung, C.T., Niemela, S.L. and Miller, R.H. (1989) One-step preparation of competent Escherichia coli: transformation and storage of bacterial cells in the same solution. *Proc Natl Acad Sci U S A,* 86, 2172-2175.
18. Hanahan, D. (1985) Techniques for transformation of *E*. *coli.* In Glover, D. (ed.), *DNA cloning.* A *practical approach.* IRL Press, Oxford, Vol. 11 pp. 109-135.
19. Hartung, M. and Kisters-Woike, B. (1998) Cre mutants with altered DNA binding properties. *J Biol Chem,* 273, 22884-22891.
20. Guo, F., Gopaul, D.N., and van Duyne, G.D. (1997) Structure of Cre recombinase complexed with DNA in a site-specific recombination synapse. *Nature,* 389, 40-46.

## Patentansprüche

1. Verwendung von zwei nicht identischen Erkennungssequenzmutanten für sequenzspezifische Rekombinasen, wobei die Erkennungssequenzmutanten jeweils zwei Erkennungssequenzen enthalten, die durch eine Spacersequenz getrennt sind, und wobei die Mutanten in jeweils einer Erkennungssequenz Mutationen tragen, die zueinander invers repetitiv sind und die jeweils andere Erkennungssequenz dem Erkennungssequenz-Wildtyp entspricht, zur Durchführung zweier oder mehrerer Rekombinationsereignisse durch eine sequenzspezifische Rekombinase in einem einzelnen genetischen System.

2. Verwendung nach Anspruch 1, wobei die Erkennungssequenzmutanten im Rahmen des Cre/loxP-Systems eingesetzt werden.

3. Verwendung nach Anspruch 2, bei der die beiden loxP-Mutanten lox 66 und lox 71 nach Seq. Id. Nr. 1 und 6 eingesetzt werden.

4. Verwendung nach Anspruch 2 und 3, bei der als Erkennungssequenzmutanten eine der in den Seq. Id. Nr. 2-5 und 7-10 angegebenen Sequenzen verwendet werden.

5. Verwendung nach Anspruch 3 und 4, wobei die Erkennungssequenzmutanten der Seq. Id. Nr. 1-5 in 5'-> 3'-Richtung von den Sequenzen ATTCC und TCTCG, die Erkennungssequenzmutanten der Seq. Id. Nr. 6-10 von den Sequenzen GCTTC und CTCTT flankiert sind.

6. Verwendung nach Anspruch 1, wobei die Erkennungssequenzmutanten im Rahmen des *Saccharomyces cerevisiae* Flp-FRT, des *Zygosaccharomyces rouxii* pSR1-, Resolvase-rfsF oder des Phagen *Mu Gin* Rekombinase-Systems eingesetzt werden.

7. Rekombinationsverfahren zur Durchführung mehrfacher Rekombinationen mittels einer Rekombinase in einem einzelnen genetischen System, das die folgenden Schritte umfasst:
a) Bereitstellung von zwei nicht identischen Erkennungssequenzmutanten für sequenz-spezifische Rekombinasen, wobei die Erkennungssequenzmutanten jeweils zwei Erkennungssequenzen enthalten, die durch eine Spacersequenz getrennt sind, und wobei die Mutanten in jeweils einer Erkennungssequenz Mutationen tragen, die zueinander invers repetitiv sind und die jeweils andere Erkennungssequenz dem Erkennungssequenz-Wildtyp entspricht, und wobei die beiden Erkennungssequenzmutanten so ausgerichtet sind, daß ihre Wildtyp-Sequenzen auf der dem Rekombinationsort zugewandten Seite liegen,
b) Induktion einer sequenzspezifischen Rekombinase zur Durchführung eines Rekombinationsereignisses, wobei eine Erkennungssequenzmutante zurückbleibt, die nicht mehr von der Rekombinase erkannt wird,
c) Wiederholen der Schritte a) + b) zur Durchführung weiterer Rekombinationsereignisse.

8. Rekombinationsverfahren nach Anspruch 7, bei dem die Rekombinase Cre eingesetzt wird und die beiden loxP-Mutanten lox 66 und lox 71 nach Seq. Id. Nr. 1 und 6 sind.

9. Verfahren nach Anspruch 7, bei dem die Rekombinase Cre eingesetzt wird und als Erkennungssequenzmutanten eine der in den Seq. Id. Nr. 2-5 und 7-10 angegebenen Sequenzen verwendet wird.

10. Verfahren nach Anspruch 8 oder 9, wobei die Erkennungssequenzmutanten der Seq. Id. Nr. 1-5 in 5'-> 3'-Richtung von den Sequenzen ATTCC und TCTCG, die Erkennungssequenzmutanten der Seq. Id. Nr. 6-10 von den Sequenzen GCTTC und CTCTT flankiert sind.

11. Rekombinationsverfahren nach einem der Anspüche 8-10, bei dem die Cre-Rekombinase durch einen Vektor kodiert wird, der in dem genetischen System exprimiert wird.

12. Verfahren nach Anspruch 7, wobei wobei die Erkennungssequenzmutanten im Rahmen des *Saccharomyces cerevisiae* Flp-FRT, des *Zygosaccharomyces rouxii* pSR1-, Resolvase-rfsF oder des Phagen *Mu Gin* Rekombinase-Systems eingesetzt werden.

13. Rekombinationsverfahren nach einem der Anspüche 7-12, bei dem das Rekombinationsereignis aus einer Insertion einer DNA-Sequenz besteht.

14. Rekombinationsverfahren nach einem der Anspüche 7-12, bei dem das Rekombinationsereignis aus einer Exzision einer DNA-Sequenz besteht.

15. Rekombinationsverfahren nach Anspruch 14, bei dem die DNA-Sequenz ein Markergen enthält.

16. Rekombinationsverfahren nach Anspruch 15, bei dem das Markergen ein Antibiotikaresistenzgen ist.

17. Rekombinationsverfahren nach Anspruch 16, bei dem das Antibiotikaresistenzgen eine Resistenz gegen Chloramphenicol, Tetracyclin oder Ampicillin verleiht.

18. Rekombinationsverfahren nach einem der Ansprüche 8-11, bei dem die nach dem ersten Rekombinationsereignis entstandene loxP-Mutante dieselbe Orientierung aufweist wie die zur Durchführung weiterer Rekombinationsereignisse bereitgestellten loxP-Mutanten.

19. Erkennungssequenzmutante, die durch eine der Nukleinsäuresequenzen von Seq. Id. Nr. 2-5 und 7-10 gekennzeichnet ist.


SEQUENZPROTOKOLL

<110> GSF-Forschungszentrum f. Umwelt und Ges. GmbH

<120> Einsatz mutierter Erkennungssequenzen für mehrfache
      aufeinander folgende Rekombinase-vermittelte
      Rekombinationen in einem genetischen System

<130> P14569

<140>
<141>

<150> DE 101 40 030.6
<151> 2001-08-16

<160> 15

<170> PatentIn Ver. 2.1

<210> 1
<211> 34
<212> DNA
<213> Künstliche Sequenz

<220>
<223> Beschreibung der künstlichen Sequenz:
      Oligonukleotid lox 66 ohne flanks

<400> 1
ataacttcgt atagcataca ttatacgaac ggta                                34


<210> 2
<211> 34
<212> DNA
<213> Künstliche Sequenz

<220>
<223> Beschreibung der künstlichen Sequenz:
      Oligonukleotid

<400> 2
ataacttcgt atagcataca ttatacgcac ggta                                34


<210> 3
<211> 34
<212> DNA
<213> Künstliche Sequenz

<220>
<223> Beschreibung der künstlichen Sequenz:
      Oligonukleotid

<400> 3
ataacttcgt atagcataca ttatacgccc ggta                                34

<210> 4
<211> 34
<212> DNA
<213> Künstliche Sequenz

<220>
<223> Beschreibung der künstlichen Sequenz:
      Oligonukleotid

<400> 4
ataacttcgt atagcataca ttataggtac cgta                                34


<210> 5
<211> 34
<212> DNA
<213> Künstliche Sequenz

<220>
<223> Beschreibung der künstlichen Sequenz:
      Oligonukleotid

<400> 5
ataacttcgt atagcataca ttatacgtac cggg                                34


<210> 6
<211> 34
<212> DNA
<213> Künstliche Sequenz

<220>
<223> Beschreibung der künstlichen Sequenz:
      Oligonukleotid lox 71 ohne flanks

<400> 6
taccgttcgt atagcataca ttatacgaag ttat                                34


<210> 7
<211> 34
<212> DNA
<213> Künstliche Sequenz

<220>
<223> Beschreibung der künstlichen Sequenz:
      Oligonukleotid

<400> 7
tagcgttcgt atagcataca ttatacgaag ttat                                34


<210> 8
<211> 34
<212> DNA
<213> Künstliche Sequenz

<220>
<223> Beschreibung der künstlichen Sequenz:
      Oligonukleotid

<400> 8
taccgttcgt atagcataca ttatacgaag ttat                                34


<210> 9
<211> 34
<212> DNA
<213> Künstliche Sequenz

<220>
<223> Beschreibung der künstlichen Sequenz:
      Oligonukleotid

<400> 9
taccgggcgt atagcataca ttatacgaag ttat                                34


<210> 10
<211> 34
<212> DNA
<213> Künstliche Sequenz

<220>
<223> Beschreibung der künstlichen Sequenz:
      Oligonukleotid

<400> 10
aatgcatgct atagcataca ttatacgaag ttat                                34


<210> 11
<211> 68
<212> DNA
<213> Künstliche Sequenz

<220>
<223> Beschreibung der künstlichen Sequenz:
      Oligonukleotid

<400> 11
gggaagcttc taccgttcgt atagcataca ttatacgaag ttatctcttg cgggatatcg 60
tccattcc                                                             68


<210> 12
<211> 67
<212> DNA
<213> Künstliche Sequenz

<220>
<223> Beschreibung der künstlichen Sequenz:
      Oligonukleotid

<400> 12

cccccgagat accgttcgta taatgtatgc tatacgaagt tatggaatgg acgatatccc 60
gcaagag 67

<210> 13
<211> 34
<212> DNA
<213> Künstliche Sequenz

<220>
<223> Beschreibung der künstlichen Sequenz:
      Oligonukleotid loxP

<400> 13
ataacttcgt atagcataca ttatacgaag ttat 34

<210> 14
<211> 44
<212> DNA
<213> Künstliche Sequenz

<220>
<223> Beschreibung der künstlichen Sequenz:
      Oligonukleotid lox 66 mit flanks

<400> 14
attccataac ttcgtatagc atacattata cgaacggtat ctcg 44

<210> 15
<211> 44
<212> DNA
<213> Künstliche Sequenz

<220>
<223> Beschreibung der künstlichen Sequenz:
      Oligonukleotid lox 71 mit flanks

<400> 15
gcttctaccg ttcgtatagc atacattata cgaagttatc tctt 44